# EUROPÄISCHE PATENTSCHRIFT

(11) **EP 3 568 055 B1**
(45) Veröffentlichungstag und Bekanntmachung des Hinweises auf die Patenterteilung: **19.10.2022**
(21) Anmeldenummer: 18703897.1
(22) Anmeldetag: 09.01.2018
(51) Int. Cl.: A61B 1/247, A61C 17/06, A61B 1/015

(54) **SPIEGELSAUGER MIT MITTELWAND**
SUCTION MIRROR HAVING A CENTRAL WALL
DISPOSITIF D'ASPIRATION À MIROIR MUNI D'UNE PAROI CENTRALE

(30) Priorität: 10.01.2017 DE 102017000105
(43) Veröffentlichungstag der Anmeldung: 20.11.2019
(73) Patentinhaber: Cleverdent Ltd., 48149 Münster (DE)
(72) Erfinder: CLASEN, Stephan, 48149 Münster (DE); KAYSER, Martin, 50968 Köln (DE)
(74) Vertreter: Patentanwälte Bauer Vorberg Kayser
(86) Internationale Anmeldenummer: PCT/EP2018/050467
(87) Internationale Veröffentlichungsnummer: WO 2018/130526

(56) Entgegenhaltungen:
- EP-A1- 2 181 643
- DE-A1-102013 110 302
- DE-B3-102012 100 119
- GB-A- 1 255 719
- US-A- 3 092 910
- US-A- 5 951 284
- US-A1- 2016 143 521

## Beschreibung

Die vorliegende Erfindung betrifft einen zahnmedizinischen Spiegelsauger zum Absaugen von Flüssigkeiten und Partikeln aus einem Mundraum eines Patienten, mit einem hohlen Grundkörper, der eine Außenfläche, eine Innenfläche, eine Längsachse, eine Anschlussöffnung und eine Ansaugöffnung aufweist.

Bei zahnmedizinischen Behandlungen ist es oftmals notwendig, anfallende Flüssigkeiten oder gelöste Partikel, beispielsweise Speichel, Spraywasser und Blut während der Behandlung abzusaugen. Auch kann Wasser, beispielsweise zum Reinigen oder nach Anwenden einer Multifunktionsspritze anfallen, das abgesaugt werden muss. Hierzu werden üblicherweise Spiegelsauger verwendet, die in der Regel aus einem röhrenförmigen Körper aus Kunststoff gebildet sind, an dessen Ende ein Schlauch befestigt ist, der wiederum mit einer Pumpe verbunden ist. Durch den Schlauch werden die störenden Flüssigkeiten und Festkörper abgeleitet.

Oftmals wird ein Spiegelsauger nicht vom behandelnden Zahnarzt oder Zahnchirurgen selbst, sondern von einer oder einem Assistenten geführt und gehalten, weil der behandelnde Zahnarzt mit der einen Hand ein Bohrwerkzeug und mit der anderen Hand einen Spiegel, über den er den zu behandelnden Bereich einsehen kann, halten muss. Nachteilig bei der beschrieben Vorgehensweise ist, dass die beiden Personen sehr eng beieinander um den zu behandelnden Bereich stehen oder sitzen müssen. Dies kann gerade dann, wenn es sich um verhältnismäßig schwierige oder feinmotorisch anspruchsvolle Eingriffe handelt, für den behandelnden Arzt als störend empfunden werden.

Aus der DE 102006048463 A1 ist ein medizinischer Spiegelsauger bekannt, bei dem die Innenfläche eine durch die Ansaugöffnung sichtbare verspiegelte Oberfläche aufweist. Die erfindungsgemäße spiegelnde Beschichtung ermöglicht es dem Benutzer, den medizinischen Spiegelsauger sowohl als Spiegelsauger zum Abführen von Flüssigkeiten und Partikeln, als auch gleichzeitig als Spiegel zu benutzen. Mit Hilfe eines solchen Spiegelsaugers ist es ihm nun möglich, die Behandlung ohne eine assistierende Person durchzuführen. Der Spiegelsauger wird also gleichzeitig als Spiegelsauger und als Spiegel verwendet. Die Grundidee der Kombination der beiden Geräte ist grundsätzlich sehr gut, allerdings sind die Einsatzmöglichkeiten und die Stabilität beschränkt.

Aus der US 3,092,910 A ist ein zahnmedizinischer Spiegelsauger gemäß des Oberbegriffs des Anspruchs 1 bekannt.

Die Aufgabe der vorliegenden Erfindung besteht darin, einen gegenüber dem bekannten Spiegelsauger verbesserten Spiegelsauger bereitzustellen. Dieser soll ebenfalls die Möglichkeit bieten den Mundraum während des Absaugens einzusehen. Insbesondere soll der Spiegelsauger stabil und für weitere Aufgabenstellungen geeignet sein.

Erfindungsgemäß wird die Aufgabe durch einen Spiegelsauger mit den Merkmalen des Patentanspruchs 1 gelöst.

Demnach weist der hohle Grundköper entlang seiner Längsachse eine Mittelwand aufweist, die den Hohlraum zumindest bereichsweise in einen ersten Hohlraumkanal und einen zweiten Hohlraumkanal unterteilt.

Die Mittelwand weist erfindungsgemäß eine Wandstärke auf, die ausgehend von einer freien Stirnkante zunächst zunimmt und dann im Wesentlichen gleich stark bleibt. Anders ausgedrückt ist die Mittelwand aus aerodynamischen Gründen konisch zulaufend in Richtung der freien Stirnkante ausgeführt.

Die beiden Hohlraumkanäle können für verschiedene Aufgabenstellungen genutzt werden. Beispielsweise kann ein Hohlraumkanal einen Druckluftanschluss aufweisen, über den Druckluft durch die den Spiegel aufweisende Ansaugöffnung ausgeblasen werden kann. Dadurch ist es für den Zahnarzt möglich, während der Behandlung Bereich im Mundraum freizublasen, ohne dazu das Werkzeug wechseln zu müssen. Vorteilhafterweise ist hierzu ein Ventil vorgesehen, über das die Druckluft zu oder abgeschaltet werden kann. Das Ventil ist vorzugsweise derart ausgeführt, dass es über einen Hebel mit einem Finger geöffnet und geschlossen werden kann. In einer besonders vorteilhaften Ausführungsvariante verjüngt sich der Hohlraumkanal, der dem Zuleiten von Druckluft dienen soll, in Richtung der Ansaugöffnung.

Weiterhin kann in einem der beiden Hohlraumkanäle ein Lichtleiter angeordnet sein, über den Licht in den Mundraum abgeleitet werden kann. Zusätzlich oder alternativ kann in diesem Hohlraumkanal auch ein Datenleiter beispielsweise für eine Kamera angeordnet sein, die im Bereich der Ansaugöffnung und hierbei ist. Die Anordnung der Leiter in einem dafür extra vorgesehenen Hohlraumkanal ist vorteilhaft, weil sie in diesem vor Flüssigkeit und dem Auftreffen von Partikeln geschützt sind.

Grundsätzlich ist auch eine Kombination denkbar, nämlich das der zusätzliche Hohlraumkanal sowohl einen Druckluftanschluss aufweist und zum Einleiten von Druckluft dient, aber auch einen Licht- unter oder Datenleiter aufweist.

Durch die Mittelwand ist der Spiegelsauger weiterhin stabiler. Die Mittelwand kann durchgängig über die gesamte Länge des Spiegelsaugers oder nur bereichsweise angeordnet sein. Grundsätzlich ist es möglich, dass die Mittelwand durch die Ansaugöffnung einsehbar ist bzw. bis an den Spiegel heranreicht.

Es hat sich gezeigt, dass die Fertigung des Spiegelsaugers dann besonders einfach ist, wenn der Spiegelsauger bzw. Grundkörper aus zwei Grundkörperteilen gebildet ist, die im Wesentlichen in Längsrichtung entlang einer Längsachse des Grundkörpers bzw. des Spiegelsaugers verlaufen. Die Grundkörperteile, die unabhängig voneinander gefertigt werden können, entsprechen dann den beiden Hohlraumkanälen. In einer besonders vorteilhaften Ausführungsvariante weisen beide Grundkörperteile einen geschlossenen Kanal auf, wobei eine Längsseite des Kanals im zusammengesetzten Zustand der beiden Grundkörperteile als Mittelwand dient. Die beiden Grundkörperteile bzw. Hohlraumkanäle werden also jeweils mit einer Längswand aneinander befestigt, sodass die beiden miteinander verbundenen Längswände die Mittelwand ausbilden.

Um die mit der nur einmaligen Benutzung verbundenen Kosten zu reduzieren, kann der erfindungsgemäße Spiegelsauger aus kostengünstigen Komponenten als Einwegsauger gefertigt sein. Insbesondere wird dann kein sonst üblicher Glasspiegel verwendet. Anstelle des Glasspiegels kann beispielsweise eine Metallscheibe, vorzugsweise aus hochpoliertem Stahl mit hoher Oberflächengüte, eine Folie oder eine spiegelnde, beispielsweise mit Chrom bedampfte Oberfläche verwendet werden. Auch ist eine Lackierung denkbar. Wesentlich ist, dass der verwendete Spiegel bzw. die spiegelnde Fläche mit geringeren Kosten als ein üblicher Glasspiegel zu fertigen ist.

Im Bereich der Ansaugöffnung bildet der Grundkörper eine möglichst plane Fläche aus, die mit einem spiegelnden Material versehen ist. Das spiegelnde Material kann beispielsweise eine auf die ebene Fläche aufgeklebte Folie sein. Alternativ ist es möglich, die plane Fläche zu verspiegeln, vorzugsweise mit Chrom zu bedampfen oder mit spiegelndem Lack zu lackieren. Somit ist die Herstellung schnell und einfach möglich, die Kosten sind gering.

Das erfindungsgemäße Spiegelelement ist vorzugsweise entweder durch eine spiegelnde Metallscheibe oder durch eine Kunststoffscheibe gebildet, die mit einer spiegelnden Folie oder einer anderweitig aufgebrachten Beschichtung versehen ist. Insbesondere kann die Beschichtung durch bedampfen, vorzugsweise mit Chrom, erzeugt werden.

In einer besonders vorteilhaften Ausführungsvariante besteht die Kunststoffscheibe aus dem gleichen Kunststoff wie der restliche Grundkörper bzw. restlichen Grundkörperteile. Dies hat den Vorteil, dass für die Herstellung nur ein einziges Material beschafft werden muss. Weiterhin ist dies für die Fertigung vorteilhaft, da unter gleichbleibenden physikalischen Bedingungen gefertigt werden kann, beispielsweise hinsichtlich Temperatur und Druck.

Der Grundkörper des Spiegelsaugers kann beispielsweise auch aus zwei verschweißten Längshälften gebildet sein, die das Spiegelelement in einer Nut halten. Das Spiegelelement ist also sozusagen zwischen den beiden Längshälften angeordnet und über seinen Außenumfang von beiden Längshälften bereichsweise umgeben.

In einer besonders vorteilhaften Ausführungsvariante ist der erfindungsgemäße Spiegelsauger aus zwei Grundkörperteilen gebildet, wobei
- das Spiegelelement in einer Spiegelaufnahme des ersten Grundkörperteils in einer Öffnung gehalten ist, deren Innenwandung eine obere Halteschulter ausbildet, die an einer Außenwandung des Spiegelelements anliegt, wobei die obere Halteschulter durch das erste Grundkörperteil ausgebildet ist und den gesamten Außenumfang des Spiegelelements umgibt,
- sich die Öffnung des ersten Grundkörperteils ausgehend von einer Grundkörperunterseite in Richtung der Ansaugöffnung verjüngt und auf ihrer der Grundkörperunterseite zugewandten Seite einen Durchmesser aufweist, der größer als der Durchmesser des Spiegelelements ist,
- die beiden Grundkörperteile derart spaltfrei miteinander verbunden sind, dass sie gemeinsam den Grundkörper einstückig ausbilden.

Erfindungsgemäß kann der der Grundkörper aus Komponenten bestehen, die miteinander verbunden, vorzugsweise verschweißt oder verklebt sind und so letzendlich eine quasi-einstückigen Spiegelsauger ausbilden. Quasi-einstückig im Sinne der Erfindung bedeutet, dass die beiden Grundkörperteile nach der Fertigung nur durch Zerstören wieder voneinander getrennt werden können. Besteht der Grundkörper aus zwei Grundkörperteilen, wird zusätzlich ein kostengünstiges Spiegelelement verwendet.

Eine wesentliche Erkenntnis besteht darin, dass ein ansprechendes Äußeres des Grundkörpers dann erreicht wird, wenn eines der beiden ersten Grundkörperteile möglichst groß und das andere Grundkörperteil möglichst klein ist und sich das kleinere Grundkörperteil nur in geringem Maße in Längsrichtung des Spiegelsaugers erstreckt. Dadurch ist die durch die Verbindungsflächen zwischen den Grundkörperteilen entstehende störende Nut oder der störenden Grad relativ kurz.

Weiterhin ist entscheidend, dass das kleinere Grundkörperteil und die Verbindung zwischen den Grundkörperteilen in einem Bereich angeordnet sind, den der behandelnde Arzt während der Behandlung nicht oder nur wenig berührt. Selbst wenn die Verbindungsflächen die Oberfläche des Spiegelsaugers negativ beeinflussen, führen sie nicht zu einer haptischen Störung.

Die Anordnung im Bereich der Rückseite des Spiegels, also an der Unterseites des Spiegelsaugers ist deshalb besonders vorteilhaft, weil dieser Bereich in der Regel beim Gebrauch des Spiegelsaugers nicht einsehbar ist. Auch nicht ertastbare, aber sichtbare Veränderungen der Oberfläche fallen somit kaum negativ auf.

Das erste Grundkörperteil bildet somit nahezu den gesamten Grundkörper des Spiegelsaugers aus, während das zweite Grundkörperteil im Wesentlichen lediglich die zum Einsetzen des Spiegels in das erste Grundkörperteil notwendige Öffnung verschließt. Bezogen auf die äußere Oberfläche hat das erste Grundkörperteil einen Flächenanteil von 80 bis 95 %, das zweite Grundkörperteil einen Flächenanteil von 5 bis 20 %.

Das Spiegelelement ist vorteilhafterweise rund, kann aber auch oval sein oder andere geeignete Formen aufweisen. Im Folgenden wird von der üblichen runden Form des Spiegelelements ausgegangen.

Um das Spiegelelement auf Dauer zuverlässig zu halten, weist das erste Grundkörperteil eine Spiegelaufnahme mit einer Öffnung zum Einsetzen des Spiegelelements auf. Das erste Grundkörperteil umgibt also seitlich den in die Öffnung eingesetzten Spiegel. Die Öffnung ist beim fertigen Spiegelsauger auf der Rückseite, also hinter dem eingesetzten Spiegel durch das zweite Grundkörperteil verschlossen. Die beiden Grundkörperteile sind miteinander verschweißt oder verklebt.

Die Sichtfläche des Spiegelelements bleibt frei und ist von vorne einsehbar. Das auf der Rückseite des Spiegelelements angeordnete zweite Grundkörperteil weist in einer vorteilhaften Ausführungsvariante insgesamt Abmessungen auf, die nur unwesentlich über die Abmessungen des Spiegelelements hinausgehen. Dadurch ist es möglich, zunächst das erste Grundkörperteil zu fertigen, das Spiegelelement dann von hinten in die freie Öffnung in das erste Grundkörperteil einzusetzen und schließlich die Öffnung mit dem zweiten Grundkörperteil von hinten zu verschließen.

Erfindungsgemäß verjüngt sich die Öffnung im ersten Grundkörperteil in Richtung einer Bodenfläche innerhalb des Grundkörpers im Bereich der Ansaugöffnung, sie ist derart ausgeführt und bemessen, dass der eingesetzte Spiegel in der Spiegelaufnahme an einer oberen Halteschulter anliegt, die in einer Innenwandung der Öffnung ausgebildet ist.

Wesentlich ist, dass die Öffnung der Spiegelaufnahme des ersten Grundkörperteils derart dimensioniert ist, dass das Spiegelelement in die Spiegelaufnahme einführbar ist. Die Öffnung weist deshalb auf ihrer der Grundkörperrückseite zugewandten Seite einen Durchmesser auf, der den Durchmesser des Spiegelelements übersteigt. Dementsprechend weist auch das zweite Grundkörperteil einen Durchmesser auf, der den Durchmesser des Spiegelelements übersteigt. Letztendlich ist die Öffnung in vertikaler Querschnittsrichtung ein konisch zulaufender Kanal, in dem das Spiegelelement von der breiteren Seite her eingesetzt wird. Diese Ausführungen beziehen sich auf eine runde Grundform des Spiegelelements, weist dieser eine andere Form auf, muss die Spiegelaufnahme derart ausgeführt sein, dass auch diese andere Form aufnehmbar ist.

In einer besonders vorteilhaften Ausführungsvariante ist das Spiegelelement in vertikalen Querschnitt im Wesentlichen trapezförmig ausgeführt, wobei der Durchmesser des Spiegelelements ausgehend von einer Spiegelfläche in Richtung der Grundkörperunterseite zunimmt. Wie bereits ausgeführt weist die Innenwandung der Öffnung des ersten Grundkörperteils einen dazu korrespondierenden vertikalen Querschnitt auf, ihr Durchmesser nimmt ausgehend von der Grundkörperunterseite zu.

Die trapezförmige Form der äußeren Wandung des Spiegelelements und der Innenwandung der Öffnung bzw. die obere Halteschulter sind vorteilhafterweise derart gewählt, dass die sichtbare Spiegeloberfläche im eingesetzten Zustand des Spiegelelements bündig mit der Bodenfläche des ersten Grundkörperteils, die die Spiegelfläche umgibt, abschließt. Die obere Halteschulter verhindert durch die Anlage an der Außenseite des Spiegelelements, dass das Spiegelelement nach oben über die Bodenfläche überstehen oder sich in diese Richtung aus dem Grundkörper lösen kann.

Vorteilhafterweise ist das Spiegelelement bereits beim Einsetzen in das Spiegelelementaufnahme reib- oder formschlüssig gehalten. Beispielsweise kann der Durchmesser der Öffnung minimal geringer ausgeführt sein, als der Durchmesser des Spiegelelements. Das Spiegelelement verformt dann beim Einsetzen das umgebende Material, drückt es etwas zurück, so dass das Spiegelelement anschließend durch das elastische Material gehalten wird. Anschließend wird das zweite Grundkörperteil an das erste Grundkörperteil mit dem eingesetzten und gehaltenen Spiegel angeklebt oder angespritzt.

Alternativ ist auch denkbar, dass nicht der gesamte Durchmesser der Öffnung geringer als der Durchmesser des Spiegelelements ist, sondern das lediglich mehrere, vorzugsweise drei über den Verlauf der Innenwandung oder Halteschulter gleichmäßig verteilte Erhöhungen vorgesehen sind, die das Spiegelelement bereits vor der Verbindung mit dem zweiten Grundkörperteil in seiner Position halten.

Die Spiegeloberfläche und die umgebende Bodenfläche bilden eine möglichst plane Gesamtfläche, über die der Luftstrom, angesaugte Flüssigkeit und Partikel optimal abgeführt werden können. Die plane Gesamtfläche bewirkt auch, dass die Geräuschentwicklung durch Luftverwirbelungen in diesem Bereich gering ist. Ein Überstand des Spiegelelements gegenüber der Bodenfläche des ersten Grundkörperteils von bis zu 0,3 mm wird im Sinne der Erfindung noch als bündig angesehen.

Alternativ zur reinen Trapezform kann das Spiegelelement etwa im Mittelbereich seines vertikalen Querschnitts einen maximalen Durchmesser aufweisen. Der Durchmesser nimmt dann also von der Spiegelelementfläche ausgehend zunächst zu und anschließend in Richtung der Rückseite des Spiegelelements wieder ab. Die Innenwandung der Öffnung ist dann korrespondierend ausgebildet, so dass das Spiegelelement in die sich ausgebildete Spiegelhalterungsnut eingeklipst werden kann. Die Innenwanddung der Öffnung bildet dann nicht nur eine obere Halteschulter, sondern auch eine untere Halteschulter aus. Denkbar ist auch, dass die untere Halteschulter, die das Spiegelelement ausgehend von seiner Rückseite noch vor seinem maximalen Durchmesser kontaktiert, durch das zweite Grundkörperteil gebildet ist.

Das zweite Grundkörperteil kann derart mit dem ersten Grundkörperteil verbunden werden und eine entsprechende Form aufweisen, dass dieses das Spiegelelement innerhalb der Öffnung unter Vorspannung gegen die obere Halteschulter drückt. Dies gewährleistet, dass das Spiegelelement sicher gehalten ist und sich auch während der Behandlung nicht bewegen kann. Die Innenwandung der Öffnung wirkt als Dichtlippe und legt sich ähnlich wie ein Simmering umlaufend an die Spiegelseitenfläche an.

Für die Fertigung eignet sich insbesondere thermoplastischer Kunststoff zum Beispiel Polypropylen oder auch Polyethylen. Durch die Hinzugabe von Zusatzstoffen kann die äußere Erscheinung des Spiegelsaugers beeinflusst werden.

Das Spiegelelement befindet sich nicht vor der Ansaugöffnung, sondern in Strömungsrichtung der angesaugten Luft im Wesentlichen hinter der Ansaugöffnung, also innerhalb des Grundkörpers. Hierdurch wird erreicht, dass das Spiegelsauger nicht durch einen vorgelagerten Spiegel verlängert wird, was die Ansaugleistung vermindern würde. Bereichsweise kann das Spielelement aber auch vor der Ansaugöffnung angeordnet sein.

Obwohl ein Verkleben der Grundkörperteile miteinander grundsätzlich möglich ist, hat es sich als besonders vorteilhaft erwiesen, die beiden Grundkörperteile miteinander zu verschweißen und keinen Klebstoff zu verwenden. Die grundsätzlich mit Klebstoff verbundenen Nachteile können dadurch vermieden werden.

Vorteilhafterweise kann das erste Grundkörperteil derart gefertigt und ausgeführt werden, dass das Spiegelelement bereits vor der Verbindung der beiden Grundkörperteile miteinander reib- oder formschlüssig im ersten Grundkörperteil gehalten ist. Dies vereinfacht die nachfolgenden Fertigungsschritte.

Die Erfindung wird anhand der nachfolgenden Figuren näher erläutert. Diese zeigen lediglich Ausführungsbeispiele, die Erfindung soll nicht auf diese beschränkt sein.

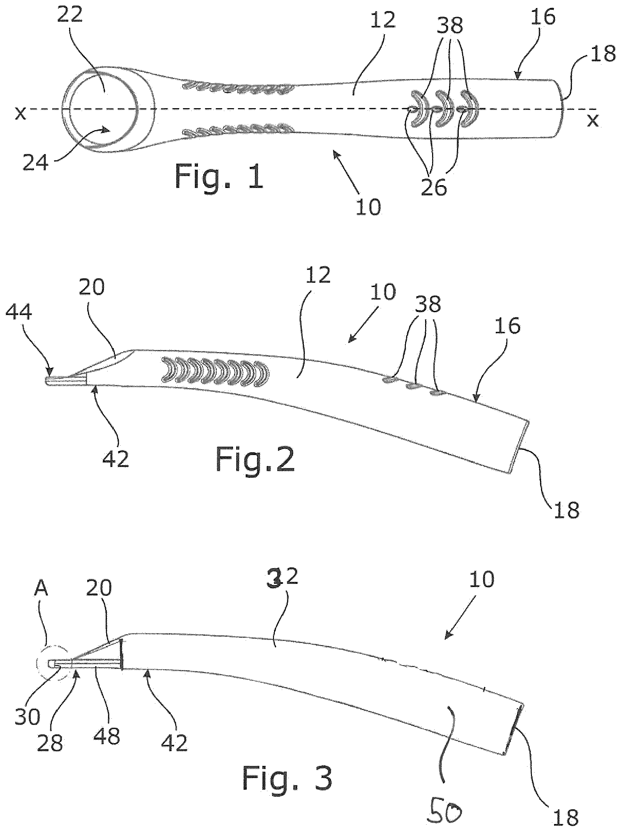

Es zeigen:
- Fig. 1:: einen erfindungsgemäßen Spiegelsauger von oben,
- Fig. 2:: den erfindungsgemäßen Spiegelsauger aus Fig. 1 von der Seite,
- Fig. 3:: einen Hohlraumkanal von der Seite in Ansicht auf eine Mittelwand,
- Fig. 4:: eine Vergrößerung des Bereichs A aus Fig. 3,
- Fig. 5.:: einen Spiegel in Seitenansicht,
- Fig. 6:: eine vereinfachte Darstellung des vorderen Bereichs des Spiegelsaugers von unten.
Wie sich insbesondere aus den Figuren 1 bis 3 ergibt, weist ein erfindungsgemäßer Spiegelsauger 10 einen hohlen rohrförmigen Grundkörper 12 mit einer Innenfläche 14 und einer Außenfläche 16 auf. Weiterhin weist der Grundkörper 12 eine Längsachse X-X auf (vergl. Fig. 1). Die insbesondere in den Figuren 2 und 3 erkennbare Bogenform des Spiegelsaugers 10 hat den Vorteil, dass dieser leichter an die zu behandelnde Stelle herangeführt werden kann.

Der Grundkörper 12 weist eine Anschlussöffnung 18 für einen nicht gezeigten Schlauch und eine Ansaugöffnung 20 zum Ansaugen von Partikeln und Flüssigkeiten auf. Durch die Ansaugöffnung 20 werden die abzusaugenden Flüssigkeiten oder Partikel eingesogen und durch die Anschlussöffnung 18 über den Schlauch abgeleitet.

Erfindungsgemäß ist innerhalb des Grundkörpers 12 ein Spiegelelement 22 im Bereich der Ansaugöffnung 20 angeordnet, das durch die Ansaugöffnung 20 einsehbar ist. Entsprechend ist eine sichtbare Spiegelfläche 24 der Ansaugöffnung 20 zugewandt. Das Spiegelelement 22 ist vollständig innerhalb des Grundkörpers 12, also in Strömungsrichtung der abzusaugende in Luft hinter der Ansaugöffnung 20 angeordnet ist. Die angesaugte Luft wird über die Spiegelfläche 24 geleitet, wodurch ein Beschlagen der Spiegelelementfläche 24 effektiv verhindert wird.

Das erfindungsgemäße Spiegelelement 22 ist vorzugsweise entweder durch eine spiegelnde Metallscheibe oder durch eine Kunststoffscheibe gebildet, die mit einer spiegelnden Folie oder einer anderweitig aufgebrachten Beschichtung versehen ist. Insbesondere kann die Beschichtung durch verdampfen, vorzugsweise mit Chrom, erzeugt werden.

Der Spiegelsauger 10 kann Zusatzöffnungen 26 aufweisen, durch die ebenfalls Luft angesaugte wird. Die Zusatzöffnungen 26 verhindern einen Unterdruck innerhalb des Grundkörpers 12, wenn die Ansaugöffnung 20 beispielsweise durch die Zunge oder Wange des Patienten verschlossen wird. Im gezeigten Ausführungsbeispiel sind drei Zusatzöffnungen 26 vorgesehen, denkbar ist aber auch nur eine einzige Zusatzöffnung 26 oder auch mehr als drei Zusatzöffnungen 26.

Auf der Außenfläche 16 des Grundkörpers 12 sind Profilelemente 38 erkennbar, die einen sicheren Griff des Spiegelsaugers 10 und ein Abrutschen der Finger des behandelnden Zahnarztes verhindern.

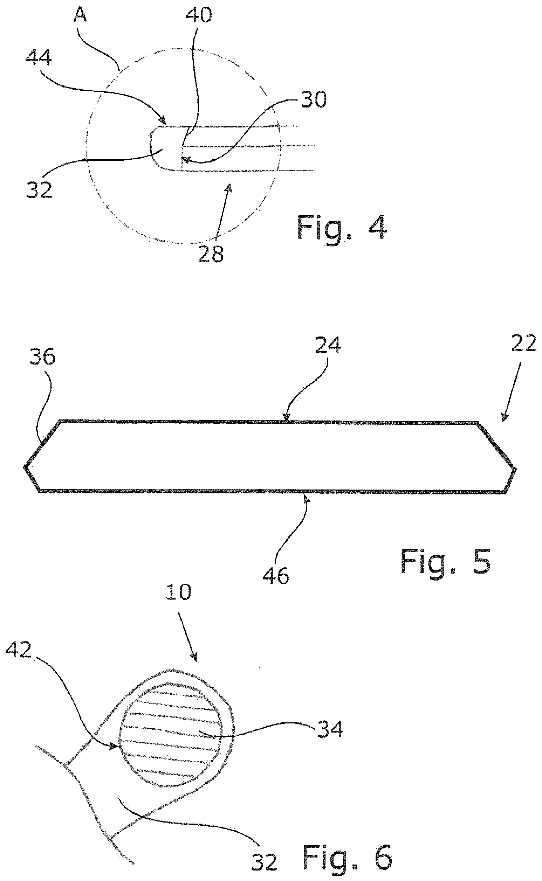

Die Figuren 3 und 6 zeigen ein Ausführungsbeispiel, bei dem der der Grundkörper 12 aus einem ersten Grundkörperteil 32 und einem zweiten Grundkörperteil 34 gebildet ist. In Figur 3 ist weiterhin eine Mittelwand 50 erkennbar. Das erste Grundkörperteil 32 weist eine Spiegelaufnahme 48 mit einer Öffnung 28 auf, in die das Spiegelelement 22 im zusammengesetzten Zustand eingesetzt ist. Die Mittelwand 50 reicht bis an das Spiegelelement 22 heran. Eine Innenwandung 30 der Öffnung 28 umgibt das Spiegelelement 22 und liegt an einer Außenwandung 36 des Spiegelelements 22 zumindest bereichsweise an. Die Öffnung 28 verjüngt sich ausgehend von einer Grundkörperunterseite 42 in Richtung der Ansaugöffnung 20. Die Öffnung 28 weist auf ihrer der Grundkörperunterseite 42 zugewandten Seite einen Durchmesser auf der größer als der Durchmesser des Spiegelelements 22 ist. Dies ist Voraussetzung dafür, dass das Spiegelelement 22 in die Spiegelaufnahme 48 einführbar ist.

Das Spiegelelement 22 ist im Querschnitt zumindest abschnittsweise etwa trapezförmig ausgeführt (vgl. Fig. 5), so dass sein Durchmesser zumindest abschnittsweise ausgehend von das Spiegelelementfläche 24 in Richtung einer Grundkörperunterseite 42 zunimmt. Das Spiegelelement 22 weist eine der sichtbaren Spiegelfläche 24 abgewandte Spiegelrückseite 46 auf. In Fig. 5 ist eine Ausführungsform gezeigt, bei der das Spiegelelement 22 in vertikaler Richtung etwa im unteren Drittel einen maschinellen Durchmesser aufweist. Diese Form vereinfacht das Einsetzen oder Einklipsen in die Spiegelaufnahme 48. An den unteren Bereich der Außenwandung 36 liegt eine untere Halteschulter an.

Fig. 4 verdeutlicht in einer vergrößerten Darstellung des Bereichs A aus Fig. 4, dass eine obere Halteschulter 40 die gesamte Außenwandung 36 des Spiegelelements 22 umgibt, und einen Raum neben und unterhalb des Spiegelelements 22 abdichtet. Die Abdichtung wird über eine Vorspannung der oberen Halteschulter 40 verbessert. Dies bedeutet, dass das Spiegelelement 22 beim Einsetzen in das erste Grundkörperteil 32 gegen die obere Halteschulter 40 gedrückt wird und diese minimal komprimiert oder elastisch verformt wird.

Weiterhin hat diese Ausführungsvariante den Vorteil, dass das Spiegelelement 22 nahezu bündig in eine Bodenfläche 44 eingelassen ist.

Fig. 6 zeigt den vorderen Bereich des Spiegelsaugers 10 mit Sicht auf die Grundkörperunterseite 42. Erkennbar ist das zweite Grundkörperteil 34 (schraffiert), welcher die Öffnung 28 verschließt.

## Patentansprüche

1. Zahnmedizinischer Spiegelsauger (10) zum Absaugen von Flüssigkeiten und Partikeln aus einem Mundraum eines Patienten, mit einem rohrförmigen hohlen Grundkörper (12), der eine Innenfläche (14), eine Außenfläche (16), eine Längsachse (X-X), eine Anschlussöffnung (18) für einen Schlauch und eine Ansaugöffnung (20) aufweist, wobei die Innenfläche (14) eine durch die Ansaugöffnung (20) einsehbare verspiegelte Oberfläche (24), aufweist, die derart angeordnet ist, dass der Mundraum über diese zumindest bereichsweise einsehbar ist, wobei der hohle Grundköper (12) entlang seiner Längsachse (X-X) eine Mittelwand (50) aufweist, die den Hohlraum zumindest bereichsweise in einen ersten Hohlraumkanal (52) und einen zweiten Hohlraumkanal (54) unterteilt, **dadurch gekennzeichnet, dass**
die Wandstärke der Mittelwand (50) ausgehend von einer freien Stirnkante (56) zunächst zunimmt und dann im Wesentlichen gleich stark bleibt.

2. Zahnmedizinischer Spiegelsauger (10) nach Anspruch 1, **dadurch gekennzeichnet, dass** die Mittelwand (50) derart ausgeführt ist, dass sich einer der beiden Hohlraumkanäle (52, 54) in Richtung der Ansaugöffnung (20) verjüngt, und sich der andere Hohlraumkanal (52, 54) in Richtung der Ansaugöffnung (20) aufweitet.

3. Zahnmedizinischer Spiegelsauger (10) nach Anspruch 2, **dadurch gekennzeichnet, dass** der sich verjüngende Hohlraumkanal (52, 54) einen Anschluss für Druckluft aufweist.

4. Zahnmedizinischer Spiegelsauger (10) nach einem der Ansprüche 1 bis 3, **dadurch gekennzeichnet, dass** in einem der beiden Hohlraumkanäle (52, 54) ein Lichtleiter verläuft ist.

5. Zahnmedizinischer Spiegelsauger (10) nach einem der Ansprüche 1 bis 4, **dadurch gekennzeichnet, dass** in einem der beiden Hohlraumkanäle (52, 54) zumindest eine Datenleitung, insbesondere für eine im Bereich der Ansaugöffnung anordbare Videokamera verläuft.

6. Zahnmedizinischer Spiegelsauger (10) nach einem der Ansprüche 1 bis 5, **dadurch gekennzeichnet, dass** die beiden Hohlraumkanäle (52, 54) voneinander unabhängig hergestellt und über die Mittelwand (50) zur Ausbildung des Spiegelsaugers (10) miteinander verbunden sind.

## Claims

1. A dental mirror suction device (10) for suctioning off liquids and particles from an oral cavity of a patient, having a hollow tubular base body (12) which has an inner surface (14), an outer surface (16), a longitudinal axis (X-X), a connection opening (18) for a hose, and a suction opening (20), wherein the inner surface (14) has a mirrored surface (24) which can be seen into through the suction opening (20) and which is disposed in a manner such that at least regions of the oral cavity can be observed by means of it, wherein the hollow base body (12) has a central wall (50) along its longitudinal axis (X-X) which divides at least regions of the hollow space into a first hollow channel (52) and a second hollow channel (54), **characterized in that** starting from a free frontal edge (56), the wall thickness of the central wall (50) initially increases and then remains at a substantially constant thickness.

2. The dental mirror suction device (10) as claimed in claim 1, **characterized in that** the central wall (50) is configured in a manner such that one of the two hollow channels (52, 54) tapers in the direction of the suction opening (20) and the other hollow channel (52, 54) widens in the direction of the suction opening (20).

3. The dental mirror suction device (10) as claimed in claim 2, **characterized in that** the tapering hollow channel (52, 54) has a connection for compressed air.

4. The dental mirror suction device (10) as claimed in one of claims 1 to 3, **characterized in that** a light guide extends in one of the two hollow channels (52, 54).

5. The dental mirror suction device (10) as claimed in one of claims 1 to 4, **characterized in that** at least one data line, in particular for a video camera which can be disposed in the region of the suction opening, extends in one of the two hollow channels (52, 54).

6. The dental mirror suction device (10) as claimed in one of claims 1 to 5, **characterized in that** the two hollow channels (52, 54) are manufactured independently of one another and connected to each other by means of the central wall (50) in order to form the mirror suction device (10).

## Revendications

1. Aspirateur dentaire à miroir (10), destiné à aspirer des liquides et des particules de la cavité buccale d'un patient, pourvu d'un corps de base (12) creux tubulaire, qui comporte une face intérieure (14), une face extérieure (16), un axe longitudinal (X-X), une ouverture de raccordement (18) pour un flexible et une ouverture d'aspiration (20), la face intérieure (14) comportant une surface réfléchissante (24) visible à travers l'ouverture d'aspiration (20), qui est placée de telle sorte que la cavité buccale soit visible au moins par endroits pas son intermédiaire, le corps de base (12) creux comportant le long de son axe longitudinal (X-X) une paroi médiane (50) qui divise la cavité au moins par endroits en une première canalisation (52) de cavité et en une deuxième canalisation (54) de cavité, **caractérisé en ce qu'**en partant d'une arête frontale (56) libre, l'épaisseur de paroi de la paroi médiane (50) augmente d'abord, pour rester ensuite sensiblement de la même épaisseur.

2. Aspirateur dentaire à miroir (10) selon la revendication 1, **caractérisé en ce que** la paroi médiane (50) est réalisée de telle sorte, que l'une des deux canalisations (52, 54) de cavité s'effile en direction de l'ouverture d'aspiration (20), et que l'autre canalisation (52, 54) de cavité s'élargisse en direction de l'ouverture d'aspiration (20).

3. Aspirateur dentaire à miroir (10) selon la revendication 2, **caractérisé en ce que** la canalisation (52, 54) de cavité qui s'effile comporte un raccord pour de l'air comprimé.

4. Aspirateur dentaire à miroir (10) selon l'une quelconque des revendications 1 à 3, **caractérisé en ce que** dans l'une des deux canalisations (52, 54) de cavité s'écoule une fibre optique.

5. Aspirateur dentaire à miroir (10) selon l'une quelconque des revendications 1 à 4, **caractérisé en ce que** dans l'une des deux canalisations (52, 54) de cavité s'écoule au moins une ligne de données, notamment pour une caméra vidéo qui peut se placer dans la zone de l'ouverture d'aspiration.

6. Aspirateur dentaire à miroir (10) selon l'une quelconque des revendications 1 à 5, **caractérisé en ce que** les deux canalisations (52, 54) de cavité sont fabriquées indépendamment l'une de l'autre et sont assemblées l'une à l'autre par l'intermédiaire de la paroi médiane (50), pour créer l'aspirateur à miroir (10).
